# EUROPEAN PATENT APPLICATION

(11) **EP 3 581 061 A1**
(43) Date of publication of application: **18.12.2019**
(21) Application number: 17895605.8
(22) Date of filing: 07.02.2017
(51) Int. Cl.: A45D 44/22

(54) **FACE MASK**

(71) Applicant: Flowfushi Co.,Ltd., Tokyo 106-0032 (JP)
(72) Inventor: KUWAJIMA, Masayuki, Tokyo 106-0032 (JP)
(74) Representative: karo IP
(86) International application number: PCT/JP2017/004453
(87) International publication number: WO 2018/146731

(57) **Abstract**

[Problem to be Solved]

To provide a beauty treatment face mask that may apply a beauty treatment action intensively to a portion of the face and may also effectively carry out a beauty treatment action throughout the entire face.

[Solution]

The face mask according to the present invention is formed of a base material sheet which has a beauty treatment action enhancement region containing a beauty treatment action enhancement substance for enhancing beauty treatment action in a portion corresponding to the facial portion where beauty treatment is especially required.

## Description

### [Technical Field]

The present invention relates to a beauty face mask.

### [Background Art]

Conventionally, a face mask for facial use formed with a base material sheet impregnated with a cosmetic composition such as a cosmetic lotion has been used for beauty treatment purposes (for example, Patent Literature 1).

Furthermore, technology has been proposed whereby metals having different polarities are embedded in the entire face mask to obtain an effect due to the weak current (for example, Patent Literature 2).

A beauty treatment face mask (pack) for covering a portion of the face has also been proposed (for example, Patent Literature 3).

### [Citation List]

### [Patent Literature]

[Patent Literature 1] JP H9-313248 A
[Patent Literature 2] JP H10-24108 A
[Patent Literature 3] JP 2005-120043 A

### [Summary of Invention]

### [Technical Problem]

With the technology whereby metal is embedded throughout a face mask covering the entire face (Patent Literature 2), facial skin sensation and user perception is dispersed throughout the entire face, preventing sufficient effective application of the cosmetic composition. In addition, since there is a greater variety of components, not only do manufacturing costs rise but it also takes time to separate the materials when disposing. Skin sensation is a sense such as touch, pain, or temperature, which is perceived as occurring on the surface of the body by being received by receptor cells present mainly in skin. In addition, it is generally known to those skilled in the art that the user's perception or awareness of the beauty treatment-targeted portion (action in the brain recognizing which portion is being applied beauty treatment) is an important element for achieving beauty treatment effect.

Although the beauty treatment face mask (pack) (Patent Literature 3) which covers a portion of face solves the problem of the above-mentioned Patent Literature 2, there is a problem that treatment of the whole face is unable to be performed.

In view of the above, the present invention provides a beauty treatment face mask capable of effectively applying a beauty treatment action to the entire face by applying a beauty treatment action to a portion of the face in a concentrated manner.

### [Solution to Problem]

A first invention is a face mask for beauty treatment formed of a base material sheet, wherein the face mask is configured so as to cover the entire user's face, and has a beauty treatment action enhancement region containing a beauty treatment action enhancement substance for enhancing beauty treatment action in a portion corresponding to a facial portion where beauty treatment is especially required.

As a result of trial and error, the inventor of the present invention found that by intensively applying beauty treatment action on a portion of the face with a face mask covering the entire face, it is possible to effectively carry out a beauty treatment action on the entire face as well. The following is considered to be the factors thereof. For example, when the relationship between a cosmetic composition impregnated in the face mask and the skin is closely observed, the action of the cosmetic composition is enhanced on the portion of the face coming into contact with the beauty treatment action enhancement region through the action of the beauty treatment action enhancement substance in the face mask of the present invention, and in addition to that, through the focusing of the user's skin perception and awareness (the above-mentioned action in the brain) on the portion of the face coming into contact with the beauty treatment action enhancement region (hereinafter referred to as "action intensive portion"), the cosmetic composition acts even more effectively in the action intensive portion. Meanwhile, even though the cosmetic composition works also in the portion of the face surrounding the action intensive portion (hereinafter referred to as the "peripheral portion"), there develops a difference in the level of action from that in the action intensive portion. Among the portions of the face, since the action intensive portion effectively absorbs the cosmetic composition and the contained amount of the cosmetic composition (hereinafter referred to as "contained amount") becomes more abundant than in the peripheral portion, a portion of the cosmetic composition in the action intensive portion seeps into the peripheral portion which has a relatively low contained amount. As a result, the peripheral portion absorbs the cosmetic composition directly from the face mask, and the cosmetic composition also seeps in from the adjacent action intensive portion. The region of the above-mentioned peripheral portion then successively expands throughout the user's face. That is, by applying the beauty treatment action intensively to one portion of the face, it is possible to effectively carry out beauty treatment action throughout the entire face. Note that in the present specification, "action enhancement" may refer to one or both of action enhancement by activation or the like of a cosmetic composition and / or action enhancement by human facial tissue activation.

A second invention is the face mask according to the configuration of the first invention, having a perception raising means for causing the user to perceive the beauty treatment action enhancement region.

According to the configuration of the second invention, the user may be made to perceive the beauty treatment action enhancement region with certainty through the perception raising means.

A third invention is the face mask according to the configuration of the second invention, wherein the perception raising means is configured so as to cause the user to perceive a region including a central portion of the beauty treatment action enhancement region.

According to the configuration of the third invention, the user may be made to perceive the central portion of the beauty treatment action enhancement region by the perception raising means.

A fourth invention is the face mask according to the configuration of the second invention or third invention, wherein the perception raising means is configured so as to raise the user to perceive only a particular portion of the beauty action enhancement region.

According to the configuration of the fourth invention, it is possible to cause the user to perceive only a particular portion of the beauty treatment action enhancement region, and not the whole. As a result, since the user's perception is focused on a narrow region, the above-mentioned intensive diffusion effect becomes even more effective.

A fifth invention is the face mask according to any one of the configurations of the first through fourth inventions, wherein the perception raising means contains a substance whose color changes according to the contained amount of a cosmetic composition contained in the base material sheet, and is configured so as to allow the user's attention to continue to be drawn by causing the user to recognize the change in color.

According to the configuration of the fifth invention, when the use of the face mask is started, the cosmetic composition is absorbed by the face of the user, and the contained amount thereof decreases. As the contained amount decreases, the color of the color change material changes. The change in color allows the user's awareness to continue to be drawn to the perception raising means. In this way, the cosmetic composition may be even more effectively applied to the portion of the face corresponding to the vicinity of the perception raising means.

A sixth invention is the face mask according to any one of the configurations of the first through fifth inventions, wherein a substance having an effect of activating the cosmetic composition and/or a blood flow promoting effect is disposed in the beauty treatment action enhancement region.

### [Advantageous Effect of Invention]

According to the face mask of the present invention, by applying the beauty treatment action to a portion of the face intensively, it is possible to effectively apply the beauty treatment action to the entire face.

### [Brief Description of Drawings]

[Figure 1] Figure 1 is a schematic plan view of a face mask according to an embodiment of the present invention.
[Figure 2] Figure 2 is a schematic side view of a face mask.
[Figure 3] Figure 3 is a schematic diagram illustrating the face of a face mask user.
[Figure 4] Figure 4 is a schematic diagram illustrating the face of a user using a face mask.
[Figure 5] Figure 5 is a conceptual diagram illustrating the movement of a cosmetic composition on a user's face.
[Figure 6] Figure 6 is a conceptual diagram illustrating the movement of a cosmetic composition in a face mask.
[Figure 7] Figure 7 is a conceptual diagram illustrating the movement of a cosmetic composition on a user's face and a face mask.
[Figure 8] Figure 8 is a schematic plan view of a face mask
according to an embodiment of the present invention.
[Figure 9] Figure 9 is a schematic side view of a face mask according to an embodiment of the present invention.
[Figure 10] Figure 10 is a schematic plan view of a face mask according to an embodiment of the present invention.
[Figure 11] Figure 11 is a schematic diagram illustrating an insertion member.
[Figure 12] Figure 12 is schematic diagram illustrating the state in which the insertion member is inserted in the face mask.

### [Description of Embodiments]

### [First Embodiment]

Preferred embodiments of the present invention will be described below with reference to the drawings. It should be noted that the description of configurations that may be appropriately implemented by those skilled in the art will be omitted and only the basic configuration of the present invention will be described.

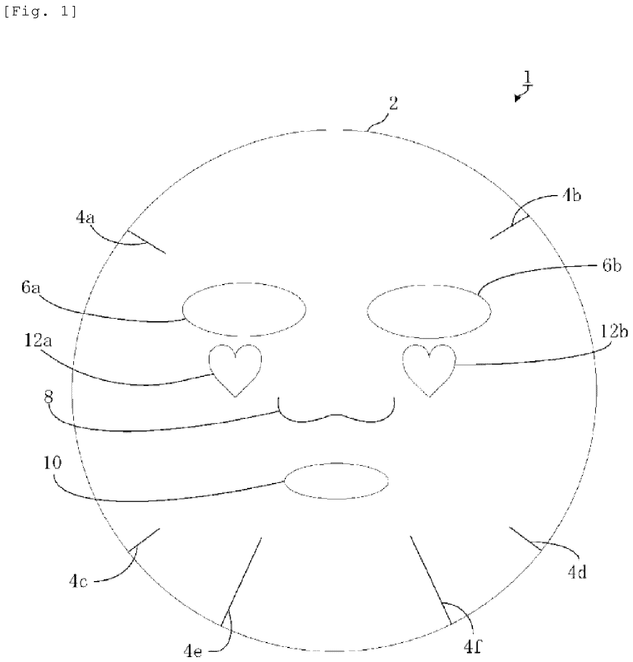

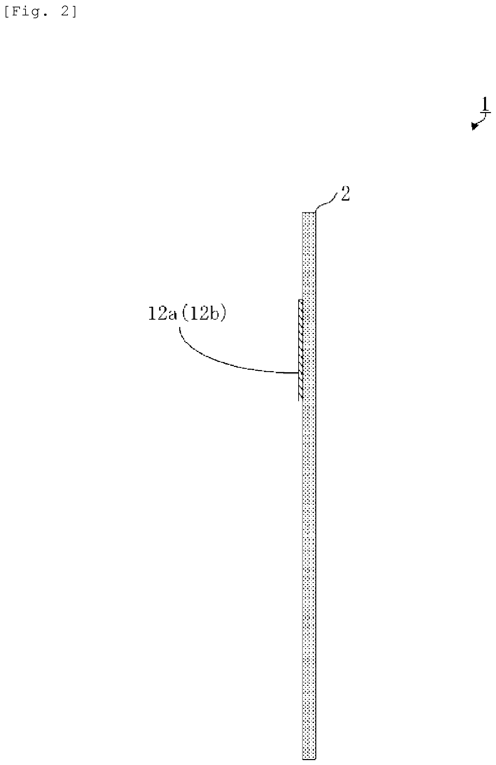

As illustrated in Figures 1 and 2, a face mask 1 is configured of a base material sheet 2. The base material sheet 2 is formed of, for example, non-woven fabric. The material of the non-woven fabric is, for example, cupro, rayon, polyethylene, polyvinyl alcohol or the like. The base material sheet 2 is impregnated with a cosmetic composition. The main component of the cosmetic composition is water, and further contains, for example, any one of the following beauty treatment components: human oligopeptide-1, human oligopeptide-13, acetylhexapeptide-8, palmitoylpentapeptide-4, water-soluble collagen, hydrolyzed collagen, succinoyl atelocollagen, magnesium ascorbyl phosphate, Saccharomyces (black sugar, placenta extract) ferment solution, sodium hyaluronate, sodium acetylated hyaluronate, sodium hyaluronate crosspolymer, cerebroside, arbutin, magnesium aspartate, dipotassium glycyrrhizate, copper gluconate, zinc gluconate, placenta extract, saitai extract, water soluble proteoglycan, platinum, sake cake extract, Iwabenkei root extract, apple fruit cultured cell extract, vitamin A oil, tocopherol, Saccharomyces cerevisiae extract, cerebroside, PCA-Na, natto gum, avocado oil, kaninabara fruit oil, apricot kernel oil, sunflower seed oil, sodium lysine dilauroyl glutamate, aminocaproic acid, or betaine.

The base material sheet 2 is configured so as to suitably cover the entire face of the user. For this purpose, the base material sheet 2 is formed with cuts 4a, 4b, 4c, 4d, 4e and 4f for conforming the base material sheet 2 to the user's facial shape. In addition, the base material sheet 2 is formed with eye openings 6a and 6b for exposing the user's eyes and a mouth opening 10 for exposing their mouth. Further, a nose slit 8 is formed in the base material sheet 2 for conforming to the shape of the user's nose.

Decorative portions 12a and 12b are formed on the base material sheet 2. The decorative portions 12a and 12b are printed with a pigment in which a substance for enhancing the action of the cosmetic composition (hereinafter, referred to as "action enhancing substance") is blended. The action enhancing substance is an example of a beauty treatment action enhancing substance. The action enhancing substance may be, for example, a substance having a blood flow promoting effect on the user's face, or a substance that activates the cosmetic composition itself. Specifically, it may be a substance that generates negative ions, such as germanium, or a substance that generates far infrared rays, such as titanium oxide. Such substances include, for example, a product named Endmineral (registered trademark). Endmineral, a substance containing natural mineral ore, has a beneficial effect on beauty treatment such as generation of negative ions, has a blood flow promoting effect, and is suitable as an action enhancement substance. The decorative portions 12a and 12b are formed on the side opposite to the side in contact with the user's face. In addition, the decorative portions 12a and 12b, when worn on the user's face, are formed at a position corresponding to a portion of the user's face, and in particular, a portion where beauty treatment is especially required. The decorative portions 12a and 12b are configured with patterns and colors different from those of the base material sheet 2 and are configured so as to draw the user's attention. The decorative portions 12a and 12b are configured so as to cause the user to perceive the region including the central portion. The decorative portions 12a and 12b are examples of a beauty treatment action enhancement region and, at the same time, examples of a perception raising means.

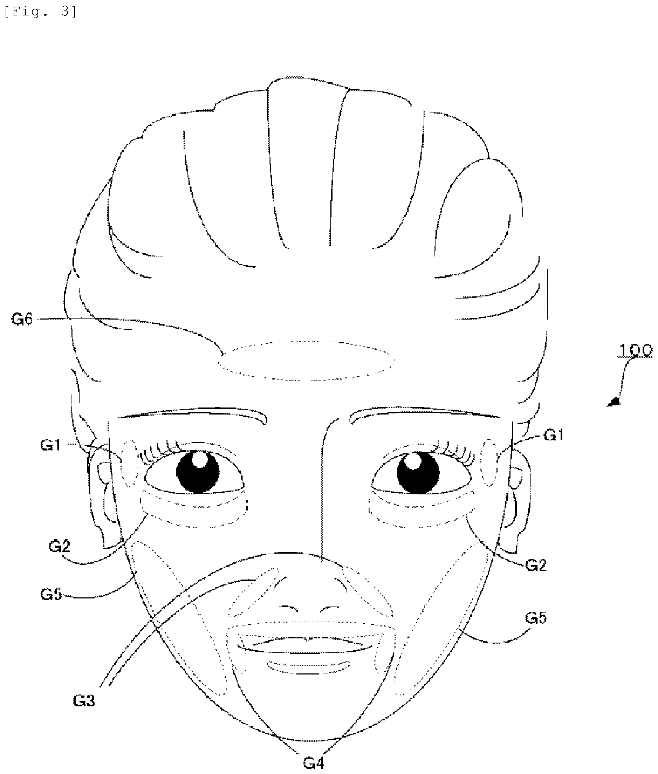

On the user's face 100, the portions especially requiring treatment are, for example as illustrated in Figure 3, the temples G1, the under eyes G2 where dark circles may be of concern, the portions G3 prone to developing nasolabial folds, the corners of the mouth G4 where wrinkles may be of concern, and the jaw area G5 where sagging may be of concern, and the forehead G6.

As illustrated in Figure 2, since the decorative portions 12a and 12b are formed on the side of the base material sheet 2 opposite to the side in contact with the user's face, when the user wears the face mask 1, the decorative portions 12a and 12b enter the user's field of vision to stimulate her/his photoreceptor cells and cause recognition of which portion the beauty treatment is being applied to. Note that the decorative portions 12a and 12b may be arranged not only in one portion corresponding to the under eyes G2, for example, but may also be arranged in a plurality of portions, such as portions corresponding to the under eyes G2 and the temples G1; however, it is not arranged throughout the entirety of the base material sheet 2.

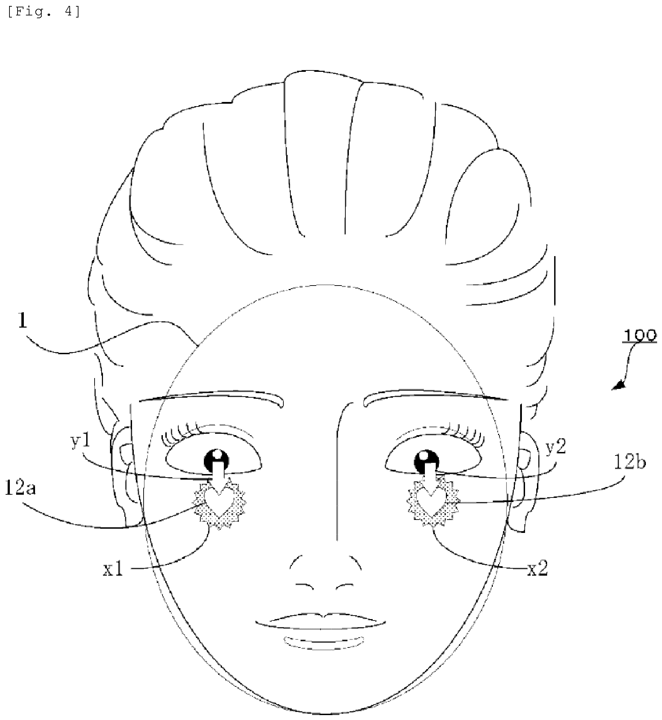

As illustrated in Figure 4, when the user wears the face mask 1, the receptor cells in the skin of the user's face 100 perceive the action of the action enhancing substance, as conceptually shown by the symbols x1 and x2. In addition, the decorative portions 12a and 12b enter the field of view of the user as shown by the arrows y1 and y2, or when looking at a mirror through the mirror, and cause the user to perceive or become aware that the portion of their face 100 in contact with the decorative portions 12a and 12b are the central regions where intensive beauty treatment is being carried out.

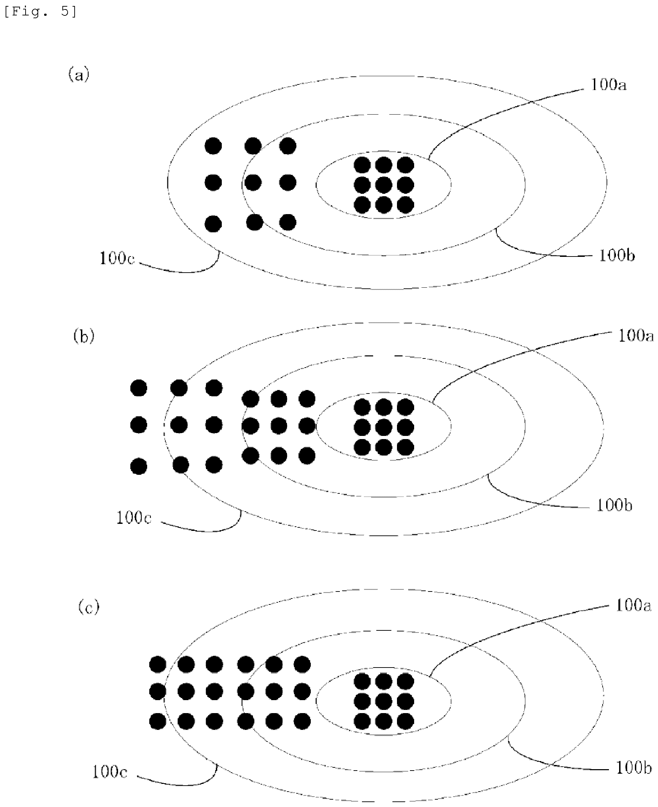

The movement of the cosmetic composition throughout the user's face 100 will be described conceptually while referencing Figure 5. The black dots in Figure 5 are a conceptual illustration of the molecules of the cosmetic composition and illustrates that the larger the density of the black dots, the higher the amount contained. A region 100a is a region in the user's face 100 in contact with the decorative portions 12a and 12b of the face mask 1, a region 100b is a region around the region 100a, and a region 100c is a region around the portion 100b.

In the first state illustrated in Figure 5(a), the region 100a has a greater contained amount than the regions 100b and 100c because it actively absorbs the cosmetic composition through the action of the action enhancement substance.

In the second state (Figure 5(b)) following the first state, the cosmetic composition in the region 100a moves to the region 100b due to the difference in contained amount between the region 100a and the region 100b. The region 100a still actively absorbs the cosmetic composition from the face mask 1 by the action of the action enhancement substance.

In the third state (Figure 5(c)) following the second state, the cosmetic composition moves from the region 100b to the region 100c due to the difference in contained amount of the region 100b and the region 100c. In the meantime, since the force homogenizing the contained amounts between the region 100a and the region 100b acts, the cosmetic composition moves from the region 100a to the region 100b. The region 100a still actively absorbs the cosmetic composition from the face mask 1.

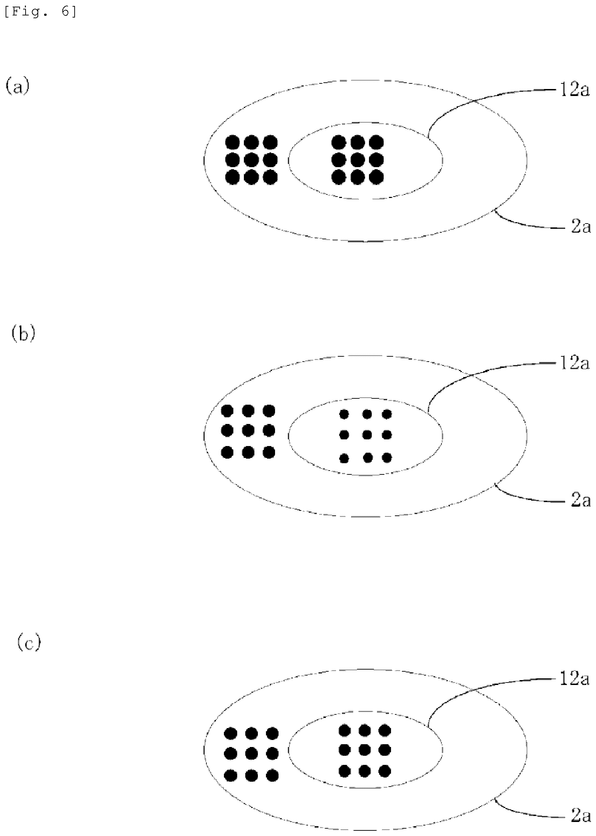

Next, the movement of the cosmetic composition throughout the face mask 1 will be described conceptually while referencing Figure 6.

The first state illustrated in Figure 6(a) is the state before being worn by the user. The contained amount in the decorative portion 12a and the contained amount in the peripheral portion 2a are sufficiently large.

In the second state (Figure 6(b)) following the first state, since the cosmetic composition in the decorative portion 12a is effectively absorbed by the user's face, the contained amount decreases relatively quickly while in the peripheral portion 2a, the contained amount decreases relatively slowly.

In the third state (Figure 6(c)) following the second state, the cosmetic composition moves from the peripheral portion 2a to the decorative portion 12a. In addition, the cosmetic composition moves from the peripheral portion (not shown) of the peripheral portion 2a to the peripheral portion 2a, and the entire face mask 1 exhibits a movement in which the contained amount is averaged-out.

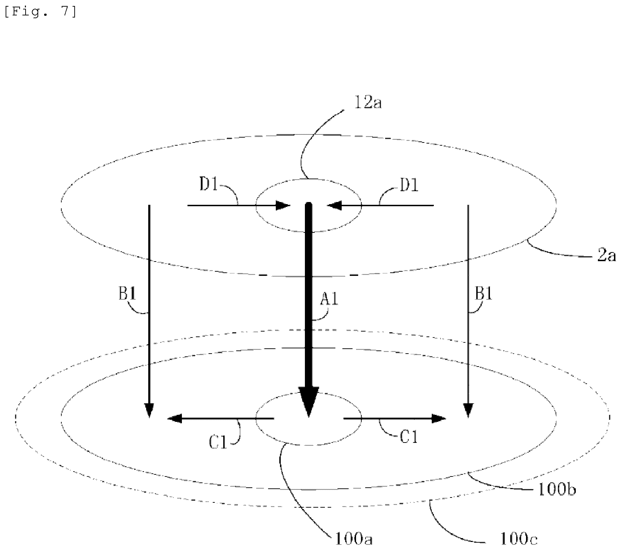

An overview of the movement of the cosmetic composition in the user's face 100 and the face mask 1 will be comprehensively described while referencing Figure 7. As illustrated by the arrow A1, the cosmetic composition is absorbed in a relatively large amount from the decorative portion 12a into the portion 100a of the face 100. Meanwhile, the cosmetic composition is absorbed in a relatively small amount from the peripheral portion 2a of the decorative portion 12a into the portion 100b of the face 100. The cosmetic composition then moves on the face 100 from the portion 100a to the portion 100b due to the osmotic pressure due to the difference in the contained amount in the portion 100a and the portion 100b, as illustrated by the arrow C1. Meanwhile, in the face mask 1, as illustrated by the arrow D1, the cosmetic composition moves from the portion 2a where the contained amount of the cosmetic composition is relatively large to the decorative portion 12a where the contained amount of the cosmetic composition is relatively small.

### [Modification]

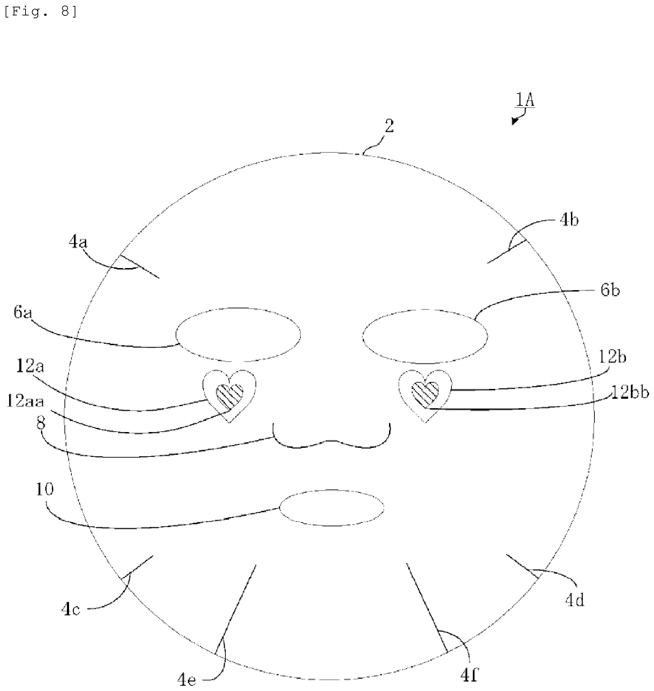

A modification will now be described using Figure 8. Note that descriptions of matters in common with the first embodiment will be omitted. In a face mask 1A of the modification, in the decorative portion 12a and the decorative portion 12b, a pattern or coloring is applied such that the central portions 12aa and 12bb are more noticeable. As a result, the user is made aware of a portion of the action enhancement region in a focused manner.

A substance whose color changes depending on the amount of water (hereinafter, referred to as "color-changing substance") may be added to the coloring ingredients of the central portions 12aa and 12bb. When the use of the face mask is started, the cosmetic composition is absorbed by the user's face and its contained amount decreases. As the contained amount decreases, the color of the color-changing substance changes, and the color of central portions 12aa and 12bb changes. The change in color allows the user's perception to continue to be drawn to the central portions 12aa and 12bb. As a result, the cosmetic composition may be more effectively applied to the face portion corresponding to the vicinity of central portions 12aa and 12bb.

In addition, if the cosmetic composition contained in the face mask is reduced or the face mask continues to be worn even after it is not present, there is a problem that develops where moisture is absorbed from the user's face to the face mask. For this reason, there is an appropriate length of time for wearing the face mask, for example, 15 minutes to 20 minutes. By including in the coloring ingredient, a substance whose color changes depending on the amount of water, when the amount of water decreases, the colors of the central portions 12aa and 12bb change. As a result, the user may be alerted to the fact that the appropriate usage time of the face mask has elapsed.

### [Second Embodiment]

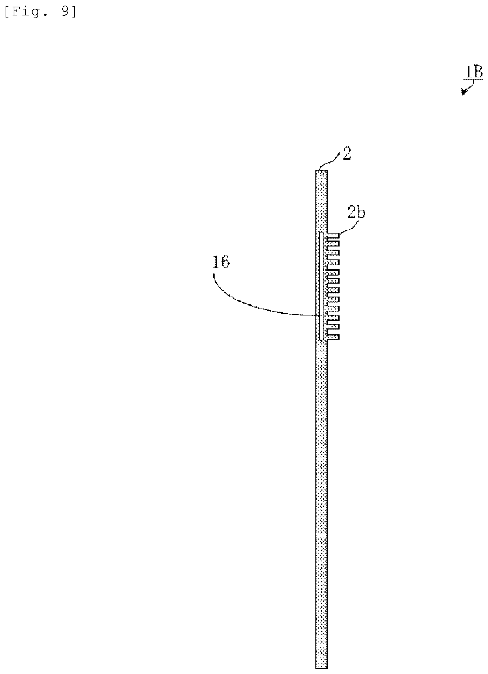

A second embodiment will now be described using Figure 9. Note that descriptions of matters in common with the first embodiment will be omitted. As illustrated in Figure 9, in a face mask 1B, projections 2b are formed on a base material sheet 2. The projections 2b are formed on the side which comes into contact with the user's face 100. In addition, an action enhancement substance 16 is disposed within the base material sheet 2. The region where the action enhancement substance 16 is disposed is an example of the action enhancement region. According to this configuration, the receptor cells of the user's face 100 wearing the face mask 1B sense the pressure from the projections 2b in addition to the action of the action enhancement substance so as to make the user aware of the region where intensive beauty treatment is being carried out. Unlike the second embodiment, the projections 2b may also be formed only in a region narrower than the region in which the action enhancement substance 16 is disposed. In addition, the action enhancement substance 16 may be disposed not within the face mask 1B but on the surface of the side not in contact with the user's face 100.

### [Third Embodiment]

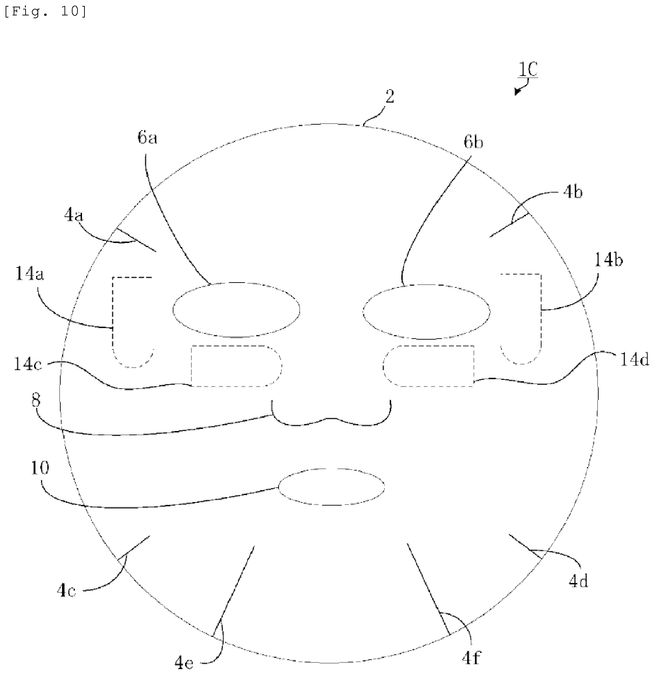

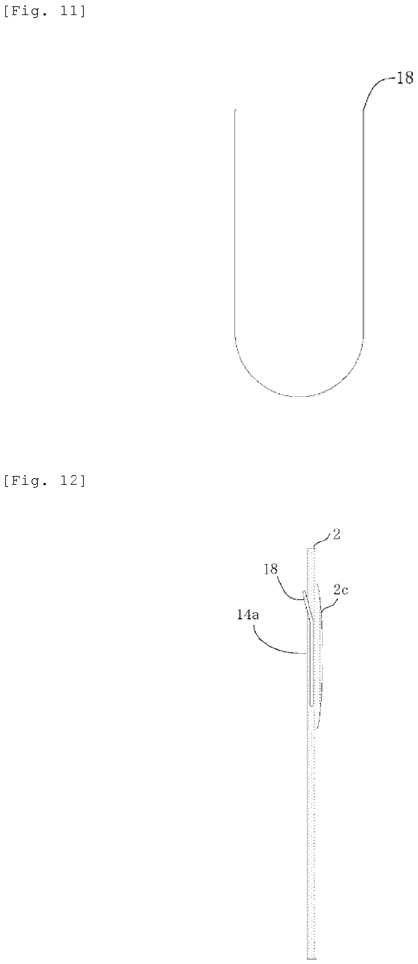

A third embodiment will now be described using Figures 10 to 12. Note that descriptions of matters in common with the first embodiment will be omitted. As illustrated in Figure 10, in a face mask 1C, pockets 14a, 14b, 14c and 14d are formed in the base material sheet 2. An insertion member 18 illustrated in Figure 11 may be inserted into the pockets 14a and the like. An action enhancement substance is disposed in the insertion member 16. The portion into which the insertion member 16 is inserted is an example of an action enhancement region. The user may select any of the pockets 14a and the like to insert the insertion member 16. As illustrated in Figure 12, for example, when the insertion member 16 is inserted into the pocket 14a, the side in contact with the user's face 100 in the base material sheet 2 becomes a raised portion 2c. According to this configuration, the receptor cells of the user's face 100 wearing the face mask 1C sense the pressure by the raised portion 2c in addition to the action of the action enhancement substance so as to make the user aware of the region where intensive beauty treatment is being carried out.

The face mask of the present invention is not limited to the above embodiments, and various changes may be made without departing from the scope of the present invention.

### [References Signs List]

- 1, 1A, 1B, 1C: Face mask
- 2: Base material sheet
- 12a, 12b: Decorative portion

## Claims

1. A face mask for beauty treatment formed of a base material sheet, wherein
the face mask
is configured so as to cover the entire user's face, and
has a beauty treatment action enhancement region containing a beauty treatment action enhancement substance for enhancing beauty treatment action in a portion corresponding to a facial portion where beauty treatment is especially required.

2. The face mask according to Claim 1, comprising a perception raising means for causing the user to perceive the beauty treatment action enhancement region.

3. The face mask according to Claim 2, wherein
the perception raising means is configured so as to cause the user to perceive a region including a central portion of the beauty treatment action enhancement region.

4. The face mask according to Claim 2 or 3, wherein
the perception raising means is configured so as to raise the user to perceive only a particular portion of the beauty action enhancement region.

5. The face mask according to any one of Claims 1 to 4, wherein
the perception raising means
contains a substance whose color changes according to the contained amount of a cosmetic composition contained in the base material sheet, and
is configured so as to allow the user's attention to continue to be drawn by causing the user to recognize the change in color.

6. The face mask according to Claim 5, wherein
a substance having an effect of activating the cosmetic composition and/or a blood flow promoting effect is disposed in the beauty treatment action enhancement region.
